(19) Europäisches Patentamt

European Patent Office

Office européen des brevets

(11) EP 1 703 275 A1

(12) **EUROPEAN PATENT APPLICATION**

(43) Date of publication:
**20.09.2006 Bulletin 2006/38**

(51) Int Cl.:
**G01N 22/00** (2006.01)

(21) Application number: **06110153.1**

(22) Date of filing: **20.02.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **18.03.2005 US 82942**

(71) Applicant: **Voith Patent GmbH**
**89522 Heidenheim (DE)**

(72) Inventors:
• **Typpo, Pekka**
**Cupertino, CA CA95014 (US)**

• **Münch, Rudolf**
**89551 Königsbronn (DE)**
• **Ischdonat, Thomas**
**89429 Bachhagel (DE)**
• **Kaufmann, Oliver**
**89522 Heidenheim (DE)**

(74) Representative: **Kunze, Klaus et al**
**Voith Paper Holding GmbH & Co. KG**
**Abteilung zjp**
**Sankt Pöltener Strasse 43**
**89522 Heidenheim (DE)**

(54) **Microwave device and process for measuring basis weight of paper or density of pulp in a paper making machine**

(57) Device and process for measuring mass per unit area of sheet products and/or density of material in a pipe. The device includes a microwave element positionable to couple high frequency electromagnetic field into a sheet to be measured, and a microwave signal generator coupled to the microwave element to generate at east one operating frequency having a frequency substantially higher than a relaxation frequency of water in said microwave element. A device for measuring an effect of the sheet on the high frequency electromagnetic field is provided, as is a device for calculating mass per unit area based upon the measured effect of the sheet on the high frequency electromagnetic field.

Fig.6

**Description**

BACKGROUND OF THE INVENTION

1. Field of the Invention

The present invention is directed to a sensor and process for measuring mass per unit area, i.e., basis weight, of sheet products, e.g., paper or board. Moreover, the present invention is directed to a sensor and process for measuring the density of pulp slurry in a process pipe.

2. Discussion of Background Information

While the determination of mass per unit area (basis weight) in sheet products, such as paper or board webs is known, the prior art procedures are generally based on either absorption or scattering of beta, gamma, or x-rays. Thus, while these procedures produce acceptable results, there is always a danger of exposure to radioactive materials or harmful x-rays.

U.S. Patent No. 4,755,678 describes simultaneously measuring moisture and basis weight using sub-millimeter wave radiation. This process is based on the measurement of the absorption (which depends directly on loss factor) of the radiation energy in the sheet. Measurement is performed using two or more frequencies where the absorption by water and by dry matter in the sheet are sufficiently different so that both weight and moisture can be calculated separately.

However, there are two drawbacks with the above-noted process. First, because the mass absorption coefficient of water is many times the absorption by cellulose fibers, the slightest error in water measurement will result in a large error in the dry weight calculation. Second, the process generates the measurement frequencies utilizing a very complicated and expensive laser system that is not very suitable for the paper machine environment.

SUMMARY OF THE INVENTION

The instant invention provides a sensor that measures the mass per unit area for a sheet product without the use of hazardous radiation. In particular, the sensor according to the instant invention uses low power microwaves.

According to the invention, the sensor is structured and arranged to create a microwave field that penetrates the measured sheet.

The microwave field penetrating the measured sheet can be created in a number of ways, e.g., striplines in close proximity of the sheet or planar resonant structures, such as straight line resonators or ring resonators. Numerous other resonant shapes can also be used. However, common features for these devices include a thin conductor strip shaped to have the desired resonance modes, and an arrangement such that the material under test on one side, e.g., a front side, and a known dielectric material or an empty space is located on an opposite side, e.g., back side. For planar resonators, the resonant frequency depends directly on propagation velocity through these structures, which in turn depends on the real component of the dielectric constant of the materials in the electromagnetic field created by these structures. All these structures have multiple resonant frequencies, so sensing can be done either with a single frequency or with a series of different frequencies.

Striplines are broadband devices. In accordance with the invention, the stripline is a thin conductor strip located on a surface of a dielectric material arranged to face the material under test. The strip has either a known dielectric material or an empty space on its back side, and the material under test either close to or on its front side. Signals at the desired frequencies are fed through the line and the propagation velocity is measured and the result is used to calculate the mass per unit area, or the density, of the material under test.

When using a microwave sensor, the dielectric properties of the measured sheet influence the microwave field and this influence depends on the dielectric constant and mass of the sheet. As is known, the dielectric constant is a complex number that includes the loss factor for the material. Thus, if the dielectric constant of the sheet is stable and known, then the use of microwaves can be used at a wide range of frequencies.

At low frequencies, e.g., up to 20 GHz, the dielectric constant of the sheet or web produced in a papermaking process, changes as the sheet progresses through the papermaking machine, i.e., becomes more dry. In this regard, in the forming section, where most of the mass of the sheet is water, the dielectric constant corresponds to that of water, which is an extremely high relative dielectric constant, i.e., approximately 80 in room temperature at low frequencies, whereas, in the reeling section at the end of production, the dielectric constant of dry paper is significantly lower, with a relative dielectric coefficient of approximately 2 and a typical density of 0.7 kg/l. Thus, at low frequencies, it is almost impossible to know the dielectric coefficient of paper accurately unless the sheet moisture is accurately known. Of course, even then the total mass measurement would be almost impossible because the effect of the solids is so small in comparison with water.

However, the present invention provides a microwave sensor for measuring basis weight of the sheet on the paper machine at various locations throughout the papermaking machine. In particular, the instant sensor finds utility in the papermaking machine in areas ranging from the forming section, where the mass of the sheet is mostly water, to the reel, where the amount of water is normally less than 10%.

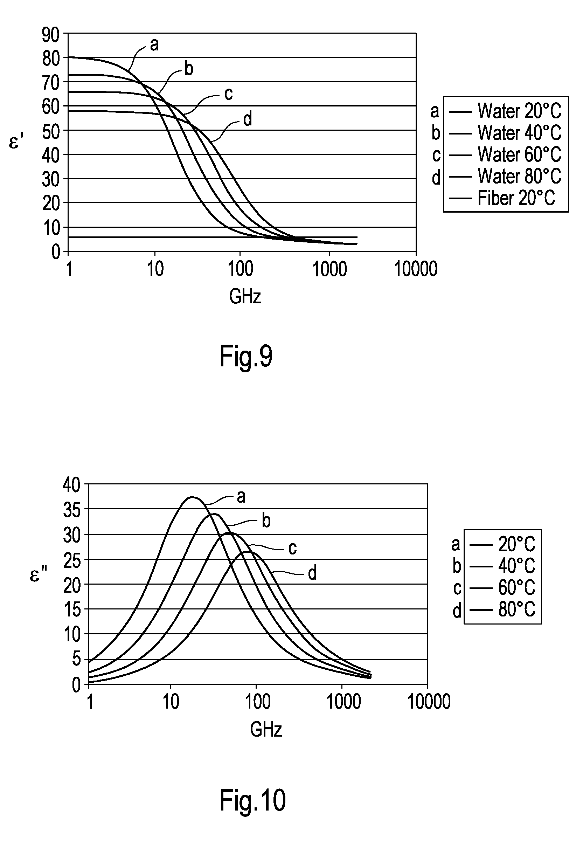

Cellulose dielectric constant will increase slightly with increasing temperature and will decrease slightly with increasing frequency. However, in the illustrated scale these changes are not visible. The dielectric constant as a function of frequency for different temperatures (a - d) is also shown in Figure 9.

Figure 10 illustrates the imaginary component (i.e., loss factor) of the dielectric coefficient of water as a function of frequency and temperature (a - d).

As the water content of the sheet in the papermaking machine will range from 99% to almost dry, it is particularly advantageous to be able to measure sheet basis weight in locations starting from the forming section to the reel of a paper machine. The inventors have found that the solution to the problem caused by the wide variation in water content in the sheet is to use very high frequencies. In this regard, when the sensor frequency is increased past the relaxation frequency of water at 22 GHz in room temperature, the dielectric constant of water will slowly drop and will ultimately reach a value of 3.51, which is less than what the wood fibers in the sheet have when their density is taken into account. Thus, it is possible to choose a frequency where the dielectric constants of water and cellulose fiber are the same.

In practice it may be more advantageous to select one frequency where the dielectric constants of all the components of the sheet are close to each other, but not necessarily the same. Moreover, the measurement can then be done with multiple frequencies. If the number of frequencies used is the same or more than the number of components in the sheet, and if the dielectric constants of the components have frequency dependencies that are sufficiently different, then the amounts of these components can be measured separately from each other. At least one of these frequencies should be significantly higher than the relaxation frequency of water, otherwise the high dielectric constant of water will prevent accurate measurement of the other components in the material under test. Typically the required frequency will be above 100 GHz.

According to the invention, the sensors for dry end basis weight measurement can be formed as either planar waveguides, such as striplines, or planar resonant structures, such as straight-line resonators or ring resonators. Moreover, the effect of the sheet on the microwave field is measured either by detecting the change in propagation velocity in the waveguide or resonant structure, rather than measuring signal attenuation. Propagation velocity depends on the real component of the dielectric constant and is virtually independent of the loss factor.

The transmission lines or resonant structures for dry end basis weight measurement can be mounted on a double sided air bearing device. Such a device can have the microwave device mounted on a surface that is held by an air bearing at a small and nearly constant distance from sheet on one side, and has a flat grounded conductor surface on the opposite side that is also separated from the sheet by an air bearing. Typical air bearing thickness is between about 100 and 200 micrometers on each side of the sheet. The total gap between the two sensor sides can be measured with a magnetic sensor, and the gap may either be held constant or measured variations in the gap size can be used to correct the measured data. Single sided measurement is also possible. However, as the distance to the sheet is critical in single sided measurement, the double sided method may be preferable. Of course, in some cases the device can be in full contact with the sheet, such that distance control is not an issue.

In the wet end of the paper machine, the sensor, which is generally located on only one side of the sheet, may require a stable sheet position. Typical application for this sensor in the wet end would be in the forming section where the sheet is supported by a plastic fabric. In such a case, the sensor can be built into or directly at one or more of the ceramic water removal elements (foils) that are in contact with the fabric. In this manner, there will be essentially no variation in the distance between the sensing element and the sheet. In a similar manner, this single sensor arrangement can be utilized in measuring density of the pulp slurry in process pipes, e.g., the sensor can be built into the pipe wall.

According to the present invention, detector arrays can be formed of a number of these sensors in order to facilitate cross machine direction and machine direction measurements of basis weight. Further, machine direction arrays can be used to determine the drainage profile in the forming section. Alternatively or additionally, the instant sensors can be used with conventional scanning applications.

In accordance with the instant invention, measurements are made using frequencies that are higher than the primary relaxation frequency of water so that the difference between the dielectric constants of water and cellulose is minimized. In this manner, it is possible to obtain an accurate total mass or density measurement. Further, as dielectric constants depend on temperature, temperature measurement may be needed to provide compensation for the measurement. If the frequency is sufficiently high and temperature variations are small, then it can be sufficient to make the measurement using just a single frequency. However, in most cases it may be preferable to use multiple frequencies, and the number of different frequencies should be either the same or higher than the number of components in the sheet.

The present invention is directed to a device for measuring mass per unit area of sheet products. The device includes a microwave element positionable to couple a high frequency electromagnetic field into a sheet to be measured, and a microwave signal generator coupled to the microwave element to generate at least one operating frequency having a frequency substantially higher than a relaxation frequency of water in said microwave element. A device for measuring an effect of the sheet on the high frequency electromagnetic field is provided, as is a device for calculating mass per unit area based upon the measured effect of the sheet on the high frequency electromagnetic field.

According to another feature of the invention, the frequency substantially higher than the relaxation frequency of water is a frequency at which the dielectric constant of water is close to that of wood fibers.

The microwave element can include a planar transmission line, and the planar transmission line can be a stripline.

The device can further include a device for measuring the effect of the sheet on propagation velocity at a single frequency. The single frequency is a frequency at which dielectric constants of water and solids in the sheet are approximately the same.

Moreover, the device may include a device for measuring the effect of the sheet on propagation velocity at a plurality of frequencies. Masses per unit area for multiple components in the sheet can be determined based upon the measured effects.

Further, the microwave element comprises a planar resonant structure, and the planar resonant structure can be one of a ring resonator and a straight-line resonator. The at least one operating frequency can be a single resonant frequency at which dielectric constants of water and solids in the sheet are approximately the same. Moreover, the device may include a device for measuring a plurality of resonant frequencies of the planar resonant structure. Masses per unit area for multiple components in the sheet can be determined based upon the measured resonant frequencies.

In accordance with another feature of the present invention, the device for calculating mass per unit area can include a measurement algorithm, and the device can further include a device for measuring temperature of the sheet. The measured temperature can be utilized as a correction in the measurement algorithm based upon temperature dependencies of dielectric constants.

According to the invention, the microwave element may be arranged for single sided measurement. The microwave element can be located in a wet section of a sheet production machine where the sheet is supported by one of a fabric or wire, and the microwave element can be located on a surface of the one fabric or wire opposite the sheet. Further, the microwave element may be located in a watering removal foil.

According to still another feature of the invention, the microwave element may be located on a first surface of the sheet and the device can also include a conducting surface located on a second surface of the sheet opposite the first surface, such that the microwave element and the conducting sheet are arranged for double sided measurement. Further, the microwave element may be located in a dry section of a sheet production machine where the sheet is unsupported. Moreover, air bearings can be arranged to separate the microwave element and the conducting surface from the sheet, and the device may also include a device for measuring a gap between the microwave element and the sheet. The measured gap can be utilized as a correction in the measurement algorithm to correct measured values.

The present invention is directed to a device for measuring density of a material in a pipe. The device includes a microwave element positionable to couple a high frequency electromagnetic field into the material in the pipe, and a microwave signal generator coupled to the microwave element to generate at least one operating frequency having a frequency substantially higher than a relaxation frequency of water in the microwave element. A device for measuring an effect of the material on the high frequency electromagnetic field is provided, as is a device for calculating density based upon the measured effect of the material on the high frequency electromagnetic field.

According to a feature of the invention, the microwave element can be a planar waveguide.

The device can also include a device for measuring the effect of the material on propagation velocity at a single frequency. The single frequency is a frequency at which dielectric constants of water and solids in the material are approximately the same.

Further, the device can include a device for measuring the effect of the material on propagation velocity at a plurality of frequencies. Masses per unit area for multiple components in the material can be determined based upon the measured effects.

In accordance with another feature of the instant invention, the microwave element may be a planar resonant structure, and the planar resonant structure comprises one of a ring resonator and a straight-line resonator. The at least one operating frequency can be a single resonant frequency at which dielectric constants of water and solids in the material are approximately the same. Further, the device can include a device for measuring a plurality of resonant frequencies of the planar resonant structure. Masses per unit area for multiple components in the material may be determined based upon the measured resonant frequencies.

The device for calculating density can include a measurement algorithm; and the device can also include a device for measuring temperature of the material. The measured temperature can be utilized as a correction in the measurement algorithm based upon temperature dependencies of dielectric constants.

The instant invention is directed to a process for measuring mass per unit area of a sheet with the device discussed above. The process includes positioning the microwave element adjacent the sheet, applying the at least one operating frequency having a frequency substantially higher than the relaxation frequency of water to the microwave element to couple a high frequency electromagnetic field to the sheet, measuring the effect of the sheet on the high frequency electromagnetic field; and calculating the mass per unit area based upon the measured effect of the sheet on the high frequency electromagnetic field.

According to a feature of the invention, the microwave element can be positioned adjacent the sheet for single sided measurement. The microwave element may be located in a wet section of a sheet production machine where the sheet is supported on one of a fabric or wire, and the process can further include positioning the microwave element on a surface of the fabric or web opposite the sheet. The process can also include locating the microwave element in or directly at a watering removal foil.

In accordance with still another feature of the present invention, the device can be positioned adjacent the sheet for double sided measurement. The microwave element may be located in a dry section of a sheet production machine where the sheet is unsupported, and the process can further include positioning the microwave element on a first surface of the sheet, and positioning a conducting surface on a second surface of the sheet opposite the first surface. The process may also include separating the microwave element and the conducting surface from the sheet with air bearings.

The present invention is directed to a process for measuring density of material in a pipe with the device discussed above. The process includes positioning the microwave element adjacent the material, applying the at least one operating frequency having a frequency substantially higher than the relaxation frequency of water to the microwave element to couple a high frequency electromagnetic field to the material, measuring the effect of the material on the high frequency electromagnetic field, and calculating the density area based upon the measured effect of the material on the high frequency electromagnetic field.

The present invention is directed to a process for measuring a parameter of fibrous material. The process includes conveying the fibrous material in a conveying direction, positioning a microwave element adjacent the conveyed fibrous material, applying at least one operating frequency having a frequency substantially higher than the relaxation frequency of water to the microwave element to couple a high frequency electromagnetic field to the conveying fibrous material, measuring the effect of the fibrous material on the high frequency electromagnetic field, and calculating one of mass per unit area and density of the fibrous material based upon the measured effect of the fibrous material on the high frequency electromagnetic field.

In accordance with still yet another feature of the present invention, mass per unit area is calculated and the fibrous material comprises a fibrous sheet. Alternatively, density is calculated and the fibrous material comprises a material in a pipe. Moreover, the microwave element can be a planar waveguide, or alternatively, it can be a planar resonant structure.

Other exemplary embodiments and advantages of the present invention may be ascertained by reviewing the present disclosure and the accompanying drawing.

BRIEF DESCRIPTION OF THE DRAWINGS

The present invention is further described in the detailed description which follows, in reference to the noted plurality of drawings by way of non-limiting examples of exemplary embodiments of the present invention, in which like reference numerals represent similar parts throughout the several views of the drawings, and wherein:

Figure 1 illustrates an exemplary embodiment of the invention;

Figure 2 illustrates the sensor as a straight-line resonator;

Figure 3 illustrates the sensor as a ring resonator;

Figure 4 illustrates an alternative embodiment of the invention;

Figure 5 illustrates the sensor located in a pipe;

Figure 6 illustrates a procedure for detecting propagation velocity of a signal;

Figure 7 illustrates a procedure for detecting phase shift of a signal;

Figure 8 illustrates a procedure for detecting resonant frequency of a resonator;

Figure 9 illustrates changes in the real component of dielectric constant of water and cellulose fiber with changing temperature and frequency; and

Figure 10 illustrates changes in the imaginary component of dielectric constant of water with changing temperature and frequency;

DETAILED DESCRIPTION OF THE PRESENT INVENTION

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the several forms of the present invention may be embodied in practice.

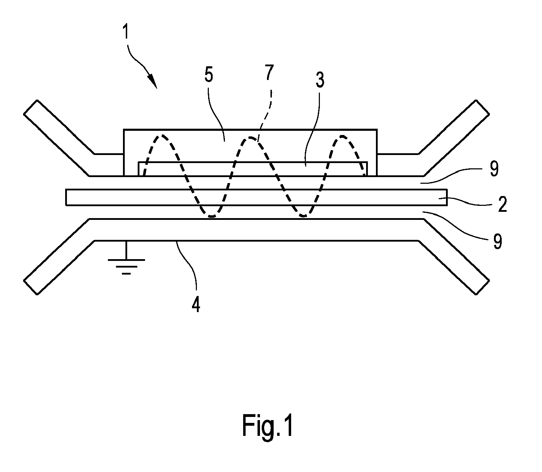

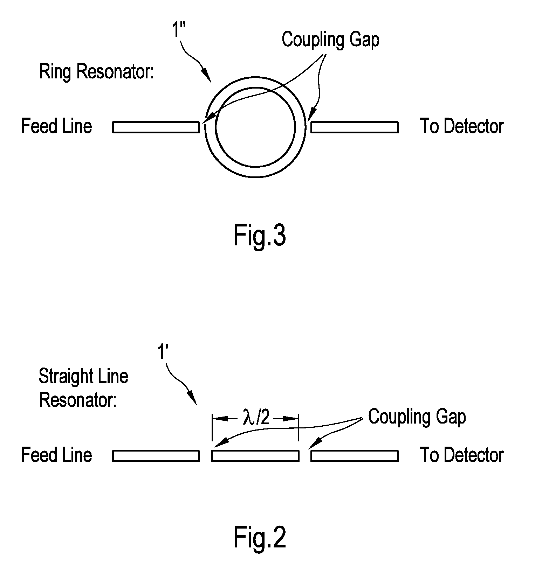

Figure 1 illustrates a sensor 1 in accordance with the features of the present invention. Sensor 1, which is preferably located in the dry end of a web or sheet production machine, e.g., a paper machine, for basis weight measurement, can be formed as a planar waveguide, e.g., a stripline, or planar resonant structures, e.g., straight-line resonators or ring resonators (see Figures 2 and 3). In particular, sensor 1 is positioned in close proximity (e.g., 0.1 mm - 0.2 mm) to sheet or web 2.

Sensor 1 can be a planar waveguide, such as a stripline, composed of a conductor 3 and a ground reference 4 separated by a dielectric layer 5, e.g., Teflon, aluminumdioxide, such that, a microwave field 7 penetrates the sheet. Conductor strip 3 is located on a surface of dielectric material 5 arranged to face the material under test. The strip has either a known dielectric material or an empty space on its back side, and sheet 2 under test either close to or on its front side. Signals at the desired frequencies are fed through the line and propagation velocity is measured. The result is used to calculate the mass per unit area, or the density, of the material under test.

Alternatively, sensor 1 can be a resonant structure, in which a resonant frequency depends on propagation velocity through the structure. Figure 2 illustrates sensor 1', which can be a straight-line resonator, and Figure 3 illustrates sensor 1", which can be a ring resonator. The resonant frequency of the planar resonant structures directly depend on propagation velocity through the structure, which depends upon the real component of the dielectric constant of the materials in the electromagnetic field created by the structures.

Thus, the effect of sheet 2 on the microwave field is measured either by detecting the change in propagation velocity in the waveguide or resonant structure, rather than measuring signal attenuation. In this regard, propagation velocity depends on the real component of the dielectric constant and is virtually independent of the loss factor.

Sensors 1, 1', or 1" can be mounted on a double sided air bearing device (not shown), such that the microwave device is mounted on a surface held by an air bearing 9 at a small and nearly constant distance (e.g., 200 $\mu$m) from the sheet on one side, and has a flat grounded conductor surface 4 on the opposite side that is also separated from the sheet by an air bearing 9. Typical air bearing thickness is between about 100 and 200 micrometers ($\mu$m) on each side of sheet 2. The total gap between the two sensor sides can be measured with a magnetic sensor, and the gap may either be held constant or measured variations in the gap size can be used to correct the measured data. In this regard, a distance sensor (not shown) can be utilized to measure a distance between sheet 2 and sensor 1, so that sensor reading corrections can be made when the distance varies or does not provide optimal readings. In this regard, a distance sensor, e.g., a magnetic or optical sensor, can also be used in a conventional manner to correct/maintain the measuring gap, e.g., by automatically adjusting air bearings 9 to control the sensor position, by automatically adjusting the position of the sensor support or sheet guides, and/or by automatically adjusting the position of sensor 1.

Moreover, measurement is also possible in which only a single side of the microwave sensor is located on one surface of the sheet without a conducting/ground surface located on the opposite surface, which is referred to hereinafter as "single sided measurement." However, as the monitoring/maintaining of the distance between the sheet and sensor in such a single sided measurement is crucial, the double sided measurement procedure, in which a conducting/ground surface is located on the sheet surface opposite the microwave sensor, are controlled or monitored may be preferable. Of course, in situations in which the microwave sensor can be in full contact with the sheet, such that distance control is not an issue and single sided measurement is facilitated.

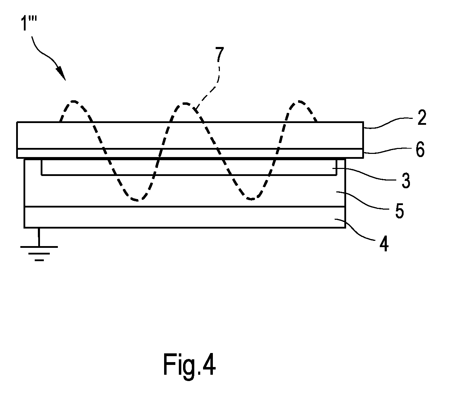

According to the instant invention, when measurement is to occur in the wet end of the sheet production machine, an exemplary arrangement is illustrated in Figure 4. In the wet end, which includes the forming section, sensor 1''' is generally only on one side of sheet 2 for a single sided measurement where a stable sheet position can be achieved. Sensor 1''', like sensor 1 in Figure 1, is composed of a conductor 3 and ground plate 4 separated by dielectric 5. However, in contrast to the dry end, sheet 2 is supported on a fabric 6, e.g., a plastic fabric or forming wire, positioned between sheet 2 and sensor 1''', which ensures essentially no spacing variation between sensor 1''' and sheet 2.

Moreover, it is noted that sensor 1''' can be built into or directly at one or more ceramic water removal elements, e.g., foils, which are in contact with fabric 6, such that no variation in spacing between sensor 1''' and sheet 2 occurs.

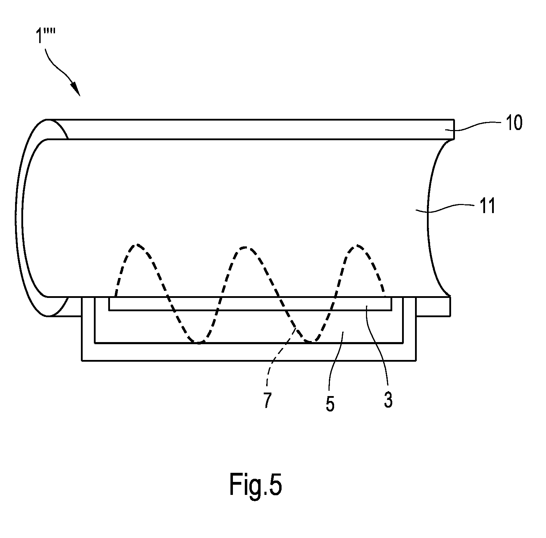

In a still further embodiment, the density of a pulp slurry in a process pipe can be measured without departing from the scope of the instant invention. In this regard, sensor 1"" can be built into the wall of a pipe 10 as illustrated in Figure 5. As shown, wall of pipe 10 houses conductor 3, which is separated from pipe 10 by dielectric 5. Moreover, microwave field 7 penetrates pulp slurry 11 within pipe 10.

According to the invention, a microwave element couples a high frequency microwave field into the sheet or slurry under test. In particular, measurements are preferably made using frequencies higher than the primary relaxation frequency of water, i.e., 22 GHz, so that differences between the dielectric constants of water and cellulose are minimized. As a result, it becomes possible to obtain an accurate total mass or density measurement. Further, as dielectric constants depend on temperature, temperature measurement may be needed to provide compensation for the measurement. If the frequency is sufficiently high and temperature variations are small, then it can be sufficient to make the measurement using just a single frequency. However, in most cases it may be preferable to use multiple frequencies, and the number of different frequencies should be either the same or higher than the number of components in the sheet.

The following discussion provides an exemplary procedure for measuring the mass per unit area of the sheet or density of the pulp slurry. Accordingly, by way of example, a model of the sensor signal according to the present invention is:

$$(1) \quad y_j = \{d - [(S_j/S0_j)^2]/[(A_j+1/d0)-A_j*(S_j/S0_j)^2]\} = \Sigma[(1-1/\varepsilon'_{ij})/\rho_i]*m_i$$

where: $S_j$ is the propagation delay (or resonant frequency if a resonator is used) measured at frequency $f_j$;
$SO_j$ is the value of S for the empty sensor measured at frequency $f_j$;
d is the size of the sensing gap;
d0 is the size of the empty sensing gap;
$A_j$ is a calibration constant at frequency $f_j$;
$\Sigma$ indicates summation over index i;
$\varepsilon'_{ij}$ is the known real component of the dielectric component of sheet component i at frequency $f_j$ at the temperature of the sheet;
$m_i$ is the mass per unit area of component i (water, fiber, $CaCO_3$, etc.); and
$\rho_i$ is the density of component i (not including the voids in the sheet, i.e. fiber density is the cell wall density).

$S_j$ and $S0_j$ are either measured with the total gap held at constant thickness or compensated for possible variations based on magnetic measurement of the gap size. Dielectric constants are modeled mathematically in software.

Mass values $m_i$ can be calculated based on equation (1) using matrix format:

$$(2) \quad M = (E*E^T)^{-1}*E^T*Y$$

where: M is a 1 x N vector with elements $m_i$, N is the number of different frequencies;
E is a K x N matrix with matrix elements $(1-1/\varepsilon'_{ij})/\rho_i$, where K is the number of components in the sheet ($N \geq K$). If N = K then $(E*E^T)^{-1}*E^T = E^{-1}$;
Y is a 1 x N vector with elements $y_j = \{d-[(S_j/S0_j)^2]/[(A_j+1/d0)-A_j*(S_j/S0_j)^2]\}$; and
Superscript T denotes matrix transposal and superscript -1 denotes matrix inversion.
The sum of the elements of vector **M** is the total weight per unit area.

When the measurement is done in a process pipe, the signal treatment and the algorithm are similar to the sheet measurement:

$$(3) \quad y_j = \{1 - [(S_j/S0_j)^2]/[(A_j+1)-A_j*(S_j/S0_j)^2]\} = \Sigma[(1-1/\varepsilon'_{ij})/\rho_i]*m_i$$

where: $S_j$ is the propagation velocity (or resonant frequency if a resonator is used) measured at frequency $f_j$;
$S0_j$ is the value of S for the empty sensor measured at frequency $f_j$;
$A_j$ is a calibration constant at frequency $f_j$;
$\Sigma$ indicates summation over index i;
$\varepsilon'_{ij}$ is the known real component of the dielectric component of component i at frequency $f_j$ at the temperature of the material in the pipe;
$m_i$ is the mass per unit volume of component i (water, fiber, $CaCO_3$, etc.); and
$\rho_i$ is the density of component i.
Mass per unit volume values $m_i$ can be calculated based on equation (1). The elements for vector Y are in this case $y_j = \{1 - [(S_j/S0_j)^2]/[(A_j+1)-A_j*(Sj/S0_j)^2]\}$ and the sum of the elements of vector **M** is the density of the fluid.

For a single sided measurement the algorithm (3) is used, the only difference is that the elements of the output vector **M** are interpreted as masses of the components per unit area.

The values for $\varepsilon'_{ij}$ can be modeled mathematically. For water a useful model is:

$$(4) \quad \varepsilon'_{water} = (\varepsilon 0\text{-}5.48)/[1+(f/f_p)^2] + 1.97/[1+(f/f_s)^2] + 3.51$$

where: f is the frequency;
fp and fs are relaxation frequencies for water; and
$\varepsilon 0$ is a model constant.

Model constant ε0 and relaxation frequencies fp and fs are temperature dependent. The temperature dependencies for these constants can be modeled with the following equations:

$$(5) \quad \varepsilon 0 = a1 - b1^*(T\text{-}273)$$

$$(6) \quad fp = exp(a2\text{-}b2/T)$$

$$(7) \quad fs = exp(a3\text{-}b3/T)$$

where: T is the absolute temperature in Kelvin; and
a1, a2, a3, b1, b2, and b3 are model constants.

Cellulose fiber dielectric constant decreases only very slightly with increasing frequency over the frequency range of interest for this invention. At constant temperature the value can be considered independent of frequency. The temperature dependence can be expressed with the following equation:

$$(8) \quad \varepsilon'_{cellulose} = 5.98 - 0.005^*(T\text{-}273)$$

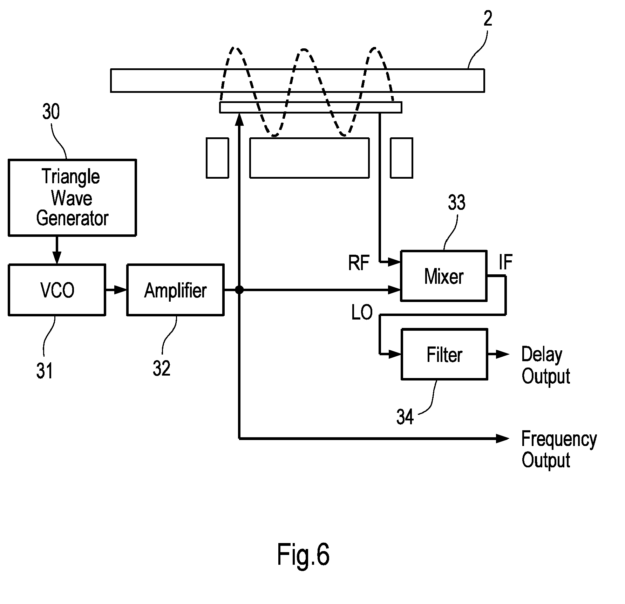

As shown in Figure 6, a frequency modulated continuation wave method can be employed to measure the effect of sheet 2 on the propagation velocity of the microwave signal through the transmission line. This method is generally used in short distance radar and microwave level detectors, and is performed by comparing a signal received at the target with a direct signal. In particular, a triangle wave generator 30 is coupled to a voltage controlled oscillator 31 in order to produce a signal frequency modulated with a triangle wave. The signal is amplified in amplifier 32 and forwarded to the microwave sensor, to mixer 33, and as a frequency output. The signal received from the microwave sensor (or the target), which has been influenced by sheet 2, is coupled to mixer 33, so that the direct and delayed signals can be compared. As the signal received from the target will be delayed slightly, the delayed signal will have a different frequency than that of the direct signal at mixer 33, so that the IF output of mixer 33 will be filtered in filter 34 and have a beat frequency proportional to the delay. In this regard, the desired propagation velocity is inversely proportional to the delay, and the delay depends upon the basis weight of sheet 2. However, the foregoing discussion of measuring propagation velocity is merely exemplary and should not be construed as limiting. Further, it is noted that any procedures for determining velocity can be utilized without departing from the spirit of the invention.

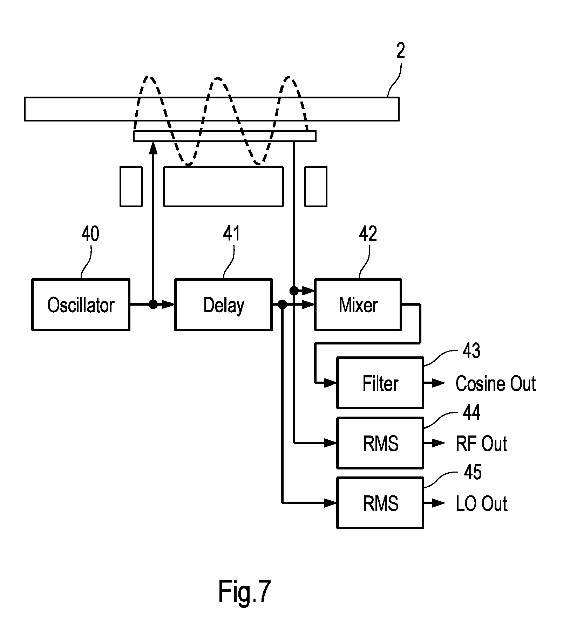

As an alternative to the frequency modulated continuation wave method discussed above, it has likewise been advantageous to calculate basis weight of sheet 2 from a measurement of phase shift of the microwave signal directed through sheet 2. As shown in Figure 7, a microwave signal from oscillator 40 is split and coupled to the microwave sensor and to delay element 41. Mixer 42, e.g., a dc-coupled balanced mixer, is arranged to receive the signal from the microwave sensor and from delay element 41 and to output a cosine out signal through filter 43. Further, RMS detector 44 is coupled to the signal from the microwave sensor to output a signal RF out, and RMS detector 45 is coupled to delay element 41 to output a signal LO out. When the signals RF and LO input to mixer 42 have exactly the same frequency, the mixer output will be proportional to the amplitudes of the input signals and the cosine of the phase angles between them. Thus, to use mixer 42 as a perfect phase detector requires that the amplitudes of RF and LO are measured and included in the calculation of phase angle. The detected phase angle can be used to calculate the propagation velocity as long as the number of wavelengths in the sensing line is known. Further, multiple oscillators at different frequencies or tunable oscillators can be switched into the system one at a time to obtain data at several frequencies. Moreover, the above-discussed procedure for measuring phase shift is provide by way of example, and should not be construed as limiting. In fact, it is noted that any procedures for determining phase shift can be utilized without departing from the spirit of the invention.

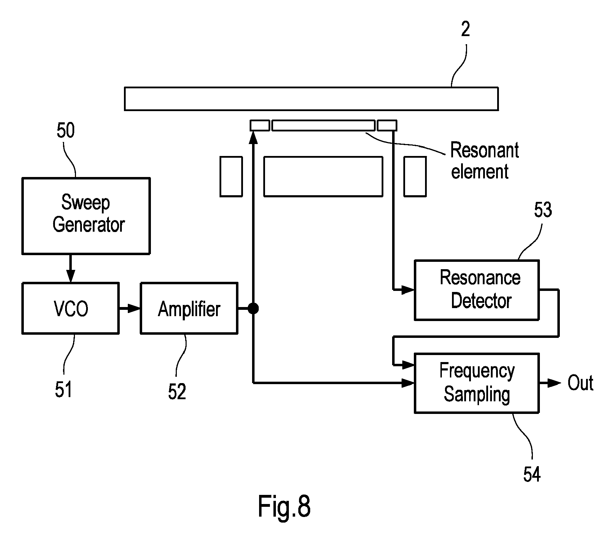

When the microwave sensor is formed as a resonator, the resonating frequency of the resonant element is measured and used to calculate the basis weight of sheet 2. While there are many ways to detect the resonant frequency of a resonant element, an exemplary procedure is illustrated in Figure 8. However, it is noted that this procedure is not intended to be limiting and that any procedures for determining resonant frequency can be utilized without departing from the spirit of the invention. An exemplary method is shown below. In the exemplary embodiment, a sweep generator 50 is coupled to a voltage controlled oscillator (VCO), which is in turn coupled to the resonant element and a frequency sampling device 54 through amplifier 52. A resonance detector 53 has an input coupled to the resonant element and an output coupled to frequency sampling device 54. Frequency sampling device 54 produces the output signal. According to this exemplary embodiment, the frequency of VCO 50 is scanned up and down over a range where resonance is to be expected. At resonance, the power from resonance detector 53 reaches a maximum, and the frequency of oscillator 50 is tracked and sampled by frequency sampling unit 54 when the derivative of the power reaches zero at a predetermined minimum power level. Frequency sweep can include several resonance modes of the resonant element. Since the frequency ranges of the oscillators may be limited, multiple sweep or tunable oscillators can be switched to the sensing system one at a time to obtain the desired frequency range.

It is noted that the foregoing examples have been provided merely for the purpose of explanation and are in no way to be construed as limiting of the present invention. While the present invention has been described with reference to an exemplary embodiment, it is understood that the words which have been used herein are words of description and illustration, rather than words of limitation. Changes may be made, within the purview of the appended claims, as presently stated and as amended, without departing from the scope and spirit of the present invention in its aspects. Although the present invention has been described herein with reference to particular means, materials and embodiments, the present invention is not intended to be limited to the particulars disclosed herein; rather, the present invention extends to all functionally equivalent structures, methods and uses, such as are within the scope of the appended claims.

## Claims

1. A device for measuring mass per unit area of sheet products, comprising:

   a microwave element positionable to couple a high frequency electromagnetic field into a sheet to be measured;
   a microwave signal generator coupled to said microwave element to generate at least one operating frequency having a frequency substantially higher than a relaxation frequency of water in said microwave element;
   a device for measuring an effect of the sheet on the high frequency electromagnetic field; and
   a device for calculating mass per unit area based upon the measured effect of the sheet on the high frequency electromagnetic field.

2. The device in accordance with claim 1, wherein the frequency substantially higher than the relaxation frequency of water is a frequency at which the dielectric constant of water is close to that of wood fibers.

3. The device in accordance with claim 1, wherein said microwave element comprises a planar transmission line.

4. The device in accordance with claim 3, wherein said planar transmission line comprises a stripline.

5. The device in accordance with claim 1, further comprising a device for measuring the effect of the sheet on propagation velocity at a single frequency, wherein the single frequency is a frequency at which dielectric constants of water and solids in the sheet are approximately the same.

6. The device in accordance with claim 1, further comprising a device for measuring the effect of the sheet on propagation velocity at a plurality of frequencies, wherein masses per unit area for multiple components in the sheet are determined based upon the measured effects.

7. The device in accordance with claim 1, wherein said microwave element comprises a planar resonant structure.

8. The device in accordance with claim 7, wherein said planar resonant structure comprises one of a ring resonator and a straight-line resonator.

9. The device in accordance with claim 7, wherein said at least one operating frequency comprises a single resonant frequency at which dielectric constants of water and solids in the sheet are approximately the same.

10. The device in accordance with claim 7, further comprising a device for measuring a plurality of resonant frequencies of said planar resonant structure, wherein masses per unit area for multiple components in the sheet are determined based upon the measured resonant frequencies.

11. The device in accordance with claim 1, wherein the device for calculating mass per unit area comprises a measurement algorithm; and said device further comprises:

    a device for measuring temperature of the sheet,

    wherein the measured temperature is utilized as a correction in said measurement algorithm based upon temperature dependencies of dielectric constants.

12. The device in accordance with claim 1, wherein said microwave element is arranged for single sided measurement.

13. The device in accordance with claim 12, wherein said microwave element is located in a wet section of a sheet production machine where said sheet is supported by one of a fabric or wire, and said microwave element is located on a surface of the one fabric or wire opposite the sheet.

14. The device in accordance with claim 12, wherein said microwave element is located in or directly at a watering removal foil.

15. The device in accordance with claim 1, wherein said microwave element is located on a first surface of the sheet and said device further comprises a conducting surface located on a second surface of the sheet opposite said first surface, such that said microwave element and said conducting sheet are arranged for double sided measurement.

16. The device in accordance with claim 12, wherein said microwave element is located in a dry section of a sheet production machine where said sheet is unsupported.

17. The device in accordance with claim 12, further comprising air bearings arranged to separate said microwave element and said conducting surface from the sheet.

18. The device in accordance with claim 17, further comprising a device for measuring a gap between said microwave element and the sheet,
    wherein the measured gap is utilized as a correction in said measurement algorithm to correct measured values.

19. A device for measuring density of a material in a pipe, comprising:

    a microwave element positionable to couple a high frequency electromagnetic field into the material in the pipe;
    a microwave signal generator coupled to said microwave element to generate at least one operating frequency having a frequency substantially higher than a relaxation frequency of water in said microwave element;
    a device for measuring an effect of the material on the high frequency electromagnetic field; and
    a device for calculating density based upon the measured effect of the material on the high frequency electromagnetic field.

20. The device in accordance with claim 19, wherein said microwave element comprises a planar waveguide.

21. The device in accordance with claim 19, further comprising a device for measuring the effect of the material on propagation velocity at a single frequency, wherein the single frequency is a frequency at which dielectric constants of water and solids in the material are approximately the same.

22. The device in accordance with claim 19, further comprising a device for measuring the effect of the material on propagation velocity at a plurality of frequencies, wherein masses per unit area for multiple components in the material are determined based upon the measured effects.

23. The device in accordance with claim 19, wherein said microwave element comprises a planar resonant structure.

24. The device in accordance with claim 23, wherein said planar resonant structure comprises one of a ring resonator and a straight-line resonator.

25. The device in accordance with claim 23, wherein said at least one operating frequency comprises a single resonant frequency at which dielectric constants of water and solids in the material are approximately the same.

26. The device in accordance with claim 23, further comprising a device for measuring a plurality of resonant frequencies of said planar resonant structure, wherein masses per unit area for multiple components in the material are determined based upon the measured resonant frequencies.

27. The device in accordance with claim 19, wherein the device for calculating density comprises a measurement algorithm; and said device further comprises:

    a device for measuring temperature of the material,

    wherein the measured temperature is utilized as a correction in said measurement algorithm based upon temperature dependencies of dielectric constants.

28. A process for measuring mass per unit area of a sheet with the device in accordance with claim 1, said process comprising:

    positioning the microwave element adjacent the sheet;
    applying the at least one operating frequency having a frequency substantially higher than the relaxation frequency of water to the microwave element to couple a high frequency electromagnetic field to the sheet;
    measuring the effect of the sheet on the high frequency electromagnetic felds and
    calculating the mass per unit area based upon the measured effect of the sheet on the high frequency electromagnetic field.

29. The process in accordance with claim 28, wherein the microwave element is positioned adjacent the sheet for single sided measurement.

30. The process in accordance with claim 29, wherein the microwave element is located in a wet section of a sheet production machine where the sheet is supported on one of a fabric or wire, and the process further comprises:

    positioning the microwave element on a surface of the fabric or web opposite the sheet.

31. The device in accordance with claim 30, further comprising locating the microwave element in a watering removal foil.

32. The process in accordance with claim 28, wherein the device is positioned adjacent the sheet for double sided measurement.

33. The process in accordance with claim 32, wherein the microwave element is located in a dry section of a sheet production machine where the sheet is unsupported, and the process further comprises:

    positioning the microwave element on a first surface of the sheet;
    positioning a conducting surface on a second surface of the sheet opposite said first surface.

34. The process in accordance with claim 33, further comprising separating the microwave element and the conducting surface from the sheet with air bearings.

35. A process for measuring density of material in a pipe with the device in accordance with claim 19, said process comprising:

    positioning the microwave element adjacent the material;
    applying the at least one operating frequency having a frequency substantially higher than the relaxation frequency of water to the microwave element to couple a high frequency electromagnetic field to the material;

measuring the effect of the material on the high frequency electromagnetic field; and
calculating the density area based upon the measured effect of the material on the high frequency electromagnetic field.

36. A process for measuring a parameter of fibrous material, said process comprising:

conveying the fibrous material in a conveying direction;
positioning a microwave element adjacent the conveyed fibrous material;
applying at least one operating frequency having a frequency substantially higher than the relaxation frequency of water to the microwave element to couple a high frequency electromagnetic field to the conveying fibrous material;
measuring the effect of the fibrous material on the high frequency electromagnetic field; and
calculating one of mass per unit area and density of the fibrous material based upon the measured effect of the fibrous material on the high frequency electromagnetic field.

37. The process in accordance with claim 36, wherein mass per unit area is calculated and the fibrous material comprises a fibrous sheet.

38. The process in accordance with claim 36, wherein density is calculated and the fibrous material comprises a material in a pipe.

39. The process in accordance with claim 36, wherein the microwave element is a planar waveguide.

40. The process in accordance with claim 36, wherein the microwave element is a planar resonant structure.

Fig.1

Ring Resonator:

1"

Coupling Gap

Feed Line

To Detector

Fig.3

Straight Line
Resonator:

1'

λ/2

Coupling Gap

Feed Line

To Detector

Fig.2

1'''

7

2

6

3

5

4

Fig.4

1""

10

11

7   5   3

Fig.5

Fig.6

Fig.7

Fig.8

Fig.9

Fig.10

**European Patent Office**

**EUROPEAN SEARCH REPORT**

Application Number

EP 06 11 0153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 297 846 A (* MMS SPACE SYSTEMS LIMITED; * MATRA MARCONI SPACE UK LIMITED) 14 August 1996 (1996-08-14)<br>* page 1, line 8 - line 11 *<br>* page 6, line 14 - page 7, line 4 *<br>* page 12, line 4 - line 6 *<br>* page 14, line 20 - page 17, line 3 *<br>----- | 1,6,28, 36,37 | INV.<br>G01N22/00 |
| D,X | US 4 755 678 A (IZATT ET AL) 5 July 1988 (1988-07-05)<br>* abstract *<br>* column 5, line 12 - line 15 *<br>----- | 1,6,28, 36,37 | |
| X | EP 0 395 308 A (PHILIP MORRIS PRODUCTS INC) 31 October 1990 (1990-10-31)<br>* abstract *<br>* column 2, line 50 - column 3, line 6 *<br>* column 5, line 1 - column 6, line 53 *<br>----- | 36 | |
| X | US 4 789 820 A (PARRENT, JR. ET AL) 6 December 1988 (1988-12-06)<br>* abstract *<br>* column 2, line 35 - line 47 *<br>* column 12, line 34 - column 13, line 32 *<br>* column 18, line 57 - line 59 *<br>* column 19, line 1 - line 21 *<br>----- | 1,2,6, 28,36,37 | TECHNICAL FIELDS SEARCHED (IPC)<br><br>G01N<br>D21F<br>D21G |
| X | US 5 886 534 A (BAKHTIARI ET AL) 23 March 1999 (1999-03-23)<br><br>* abstract *<br>* column 1, line 15 - line 32 *<br>* column 4, lines 19,50 *<br>* column 6, line 58 - line 62 *<br>----- | 1,2,5,6, 15,16, 28,32, 33,36,37 | |

-/--

~~The present search report has been drawn up for all claims~~

| Place of search | Date of completion of the search | Examiner |
|---|---|---|
| Munich | 10 July 2006 | Strohmayer, B |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**European Patent Office**

## EUROPEAN SEARCH REPORT

Application Number

EP 06 11 0153

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 30 21 096 A1 (ELEKTRO-PHYSIK HANS NIX & DR.-ING.E. STEINGROEVER KG) 10 December 1981 (1981-12-10) * page 6, lines 8,9 from bottom * * page 7, lines 2,3 * ----- | 1,12-14, 28-31 | |
| P,L, X | EP 1 624 298 A (VOITH PAPER PATENT GMBH) 8 February 2006 (2006-02-08) * the whole document * ----- | 1,2, 12-14, 28-31 | |
| X | EBBE NYFORS AND PERTTI VAINIKAINEN: "INDUSTRIAL MICROWAVE SENSORS" 1989, INDUSTRIAL MICROWAVE SENSORS , NORWOOD, US , XP002334964 ISBN: 0-89006-397-4 * page 84, paragraph 3 * * chapter 3.3.4 Strip Resonators * * page 186 - page 187 * * page 194 - page 195 * * page 224 - page 226 * ----- | 1,2,5,6, 12-16, 28-33, 36,37 | |
| X | PATENT ABSTRACTS OF JAPAN vol. 015, no. 033 (P-1158), 25 January 1991 (1991-01-25) -& JP 02 272349 A (NIPPON TOKUSHU KEISOKKI SEISAKUSHO:KK), 7 November 1990 (1990-11-07) * abstract; figures 1-4 * ----- | 1,2,5, 12-14, 28-31, 36,37 | TECHNICAL FIELDS SEARCHED (IPC) |
| X | US 4 890 054 A (MAENO ET AL) 26 December 1989 (1989-12-26) * abstract; figures 1-4,6,7 * * column 1, line 19 - line 21 * * column 1, line 39 - line 44 * ----- -/-- | 1,2,5, 15,16, 28,32, 33,36,37 | |

~~The present search report has been drawn up for all claims~~

| Place of search | Date of completion of the search | Examiner |
|---|---|---|
| Munich | 10 July 2006 | Strohmayer, B |

EPO FORM 1503 03.82 (P04C01)

**European Patent Office**

**EUROPEAN SEARCH REPORT**

Application Number

EP 06 11 0153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 214 467 A (MICROWAVE INSTRUMENTS LIMITED) 2 December 1970 (1970-12-02)<br><br>* the whole document * | 1,2,5, 15-17, 28, 32-34, 36,37 | |
| A | US 5 071 514 A (FRANCIS ET AL) 10 December 1991 (1991-12-10) * column 7, line 33 - line 43; figure 1 * | 12-16 | |

TECHNICAL FIELDS SEARCHED (IPC)

~~The present search report has been drawn up for all claims~~

| Place of search | Date of completion of the search | Examiner |
|---|---|---|
| Munich | 10 July 2006 | Strohmayer, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................

& : member of the same patent family, corresponding document

EPO FORM 1503 03.82 (P04C01)

European Patent

Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1,2,5,6,12-18,28-34,36,37

☐ None of the further search fees have been paid within the fixed time limit. The present European search report has been drawn up for those parts of the European patent application which relate to the invention first mentioned in the claims, namely claims:

European Patent
Office

LACK OF UNITY OF INVENTION
SHEET B

Application Number

EP 06 11 0153

The Search Division considers that the present European patent application does not comply with the requirements of unity of invention and relates to several inventions or groups of inventions, namely:

1. claims: 2,5 and not novel claims 1,6,28,36,37

   At the operating frequency the dielectric constant of water is close to that of wood fibers.
   Problem: measurement at a single frequency can be used to determine the total mass for a sheet consisting of water and wood fibers (application, paragraph [0064])
   ---

2. claims: 3,4,7-10,39,40

   The microwave element is planar.
   Problem: alternative microwave element
   ---

3. claim: 11

   A device for measuring the temperature of the sheet.
   Problem: temperature compensation
   ---

4. claims: 12-18,29-34

   Single sided measurement with or without conducting surface on the opposing surface.
   Problem: to enable measurement at positions, where the sheet is only accessable from one side
   ---

5. claims: 19-27,35,38

   The density of a substance in a pipe is measured.
   Problem: alternative application of the measurement method
   ---

## ANNEX TO THE EUROPEAN SEARCH REPORT
## ON EUROPEAN PATENT APPLICATION NO.

EP 06 11 0153

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2006

| Patent document cited in search report | | Publication date | Patent family member(s) | | Publication date |
|---|---|---|---|---|---|
| GB 2297846 | A | 14-08-1996 | NONE | | |
| US 4755678 | A | 05-07-1988 | NONE | | |
| EP 0395308 | A | 31-10-1990 | JP | 3128446 A | 31-05-1991 |
| | | | NO | 901804 A | 26-10-1990 |
| | | | US | 4942363 A | 17-07-1990 |
| US 4789820 | A | 06-12-1988 | AU | 589729 B2 | 19-10-1989 |
| | | | AU | 6703386 A | 09-07-1987 |
| | | | BR | 8700054 A | 01-12-1987 |
| | | | CA | 1264353 A1 | 09-01-1990 |
| | | | DE | 3688886 D1 | 16-09-1993 |
| | | | DE | 3688886 T2 | 25-11-1993 |
| | | | EP | 0240610 A2 | 14-10-1987 |
| | | | FI | 870047 A | 09-07-1987 |
| US 5886534 | A | 23-03-1999 | NONE | | |
| DE 3021096 | A1 | 10-12-1981 | NONE | | |
| EP 1624298 | A | 08-02-2006 | US | 2006028213 A1 | 09-02-2006 |
| JP 02272349 | A | 07-11-1990 | NONE | | |
| US 4890054 | A | 26-12-1989 | EP | 0292571 A1 | 30-11-1988 |
| | | | WO | 8804423 A1 | 16-06-1988 |
| | | | JP | 5075264 B | 20-10-1993 |
| | | | JP | 63145951 A | 18-06-1988 |
| GB 1214467 | A | 02-12-1970 | DE | 1598834 A1 | 15-04-1971 |
| | | | GB | 1187963 A | 15-04-1970 |
| | | | US | 3488858 A | 13-01-1970 |
| US 5071514 | A | 10-12-1991 | NONE | | |

EPO FORM P0459

For more details about this annex : see Official Journal of the European Patent Office, No. 12/82

**EP 1 703 275 A1**

**REFERENCES CITED IN THE DESCRIPTION**

**Patent documents cited in the description**

- US 4755678 A